(19)

**Europäisches Patentamt**
**European Patent Office**
**Office européen des brevets**

(11) **EP 4 685 488 A1**

(12) **EUROPEAN PATENT APPLICATION**

(43) Date of publication:
**28.01.2026 Bulletin 2026/05**

(21) Application number: **24190080.2**

(22) Date of filing: **22.07.2024**

(51) International Patent Classification (IPC):
***G01N 33/574*** *(2006.01)* ***G01N 33/68*** *(2006.01)*

(52) Cooperative Patent Classification (CPC):
**G01N 33/57407; G01N 33/6848**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **IMBA-Institut für Molekulare Biotechnologie GmbH**
  **1030 Wien (AT)**
- **Medizinische Universität Wien**
  **1090 Wien (AT)**

(72) Inventors:
- **JONSSON, Gustav**
  **1030 Wien (AT)**
- **FONTES OLIVEIRA, Tiago**
  **1110 Wien (AT)**
- **PENNINGER, Josef**
  **1130 Wien (AT)**

(74) Representative: **SONN Patentanwälte GmbH & Co KG**
  **Riemergasse 14**
  **1010 Wien (AT)**

Remarks:
•The complete document including Reference Table(s) and the Sequence Listing(s) can be downloaded from the EPO website
•The references to the drawing no. 4G is deemed to be deleted (Rule 56(4)(6) EPC).

(54) **METHOD FOR RENAL CANCER DETECTION**

(57) The invention provides a method of detecting renal cancer markers in a biological sample from a patient suspected of or to be evaluated for having renal cancer, comprising detecting one or more marker proteins selected from Lipocalin 15, Serum amyloid A1, and Haptoglobin, in the biological sample. The invention further provides a kit suitable for use with the inventive method.

**EP 4 685 488 A1**

**Description**

The present invention relates to the diagnosis of renal cancer.

**Background**

Renal cell carcinomas (RCCs) are a heterogenous group of cancers arising from the proximal convoluted tubule of the kidney. Renal cell carcinomas can be split into predominately three different histological subtypes; clear cell renal cell carcinoma (ccRCC), papillary renal cell carcinoma (pRCC) and chromophobe renal cell carcinoma (chRCC). Out of these three subtypes, ccRCC is the most common, making up approximately 80% of all RCC cases, and also the deadliest.

In 2020, approximately 431000 new cases of renal cell carcinomas were diagnosed worldwide. With a growing incidence rate, there is increased interest in cost-effective and sensitive population-wide screening programs. Despite the high clinical interest, there are no validated biomarkers for easy clinical screening, and common diagnostic procedures, such as computed tomography, are not suitable for population-based screenings (Diana et al., World journal of urology, 2023, 41(4): 929-940).

Clear cell renal cell carcinoma (ccRCC) is the renal cell malignancy with the highest incidence and mortality rates. ccRCC is primarily driven by loss-of-function mutations or epigenetic silencing of the Von Hippel landau (VHL) tumor suppressor, leading to the subsequent initiation of a hypoxia induced genetic program which drives tumor progression. Despite this clearly identified mechanism, solely screening for VHL mutations for disease detection is not sufficient due to the fact that not all patients present with clear VHL mutations. Furthermore, multiple additional co-drivers have also been identified, such as PBRM1, SETD2, KDM5C, and BAP1, making screening based on a single marker challenging (Sato et al., Nature genetics, 2013, 45(8): 860-867; Hakimi et al., European urology, 2013, 63(5): 848-854).

Differences in urine protein content has been found amongst ccRCC patients depending on disease prognosis (Sandim eet al., Urologic Oncology: Seminars and Original In-vestigations 34(1) 2016: 5.e11-5.e25) and venous infiltration (Chinello et al., Journal of proteomics, 2019, 191: 29-37). Furthermore, other attempts have been made at identifying diagnostic and prognostic protein biomarkers in the urine of ccRCC patients (Yang et al., Frontiers in Oncology, 2023, 13: 1170567; Di Meo et al., International journal of cancer, 2020, 146(8): 2315-2325). However, the performance was lower, highlighting the need for further elucidation of diagnostic biomarkers to generate a large panel of predictors.

Despite recent significant advances in the treatment of ccRCC using small molecule inhibitors and immunotherapy, early screenable markers for diagnosis are still lacking and most patients are diagnosed through computed tomography when a tumor is already suspected, for example due to palpable renal masses or hematuria (Gray and Harris, American family physician, 2019, 99(3): 179-184). Hematuria is a very common sign of ccRCC, and other renal cell malignancies, but in and of itself far from sufficient for a reliable diagnosis. Despite the relatively high patient burden compared to other renal malignancies, there are no validated biomarkers for rapid diagnosis and public health surveillance.

It is a goal of the present invention to provide new easily accessible biomarkers capable of diagnosing renal cancer such as ccRCC, preferably at an early stage.

**Summary of the Invention**

The invention provides a method of detecting renal cancer markers in a biological sample from a patient suspected of or to be evaluated for having renal cancer comprising detecting one or more marker proteins selected from Lipocalin 15, Serum amyloid A1, and Haptoglobin, in the biological sample.

The invention further provides a kit suitable with the inventive method. In particular provided is a kit comprising marker proteins or marker protein fragments, wherein the marker proteins are Lipocalin 15, Serum amyloid A1, and Haptoglobin; and wherein the marker protein fragments comprise fragments from marker proteins Lipocalin 15, Serum amyloid A1, and Haptoglobin.

Further embodiments are described in the detailed description, with each embodiment being combinable with other embodiments. The combination of all embodiments is possible. The kit can be used in the inventive method. A description of a method also reads on a suitability of the kit, which can be suitable to be used the described method. Also, combinations with any specific parameter or element described in even more detail in the examples or figures is possible.

**Detailed description of the invention**

The invention provides a method of detecting renal cancer markers in a biological sample from a patient suspected of or to be evaluated for having renal cancer. The inventive method comprises detecting one or more marker proteins selected from Haptoglobin (HP), Serum amyloid A1 (SAA1), and Lipocalin 15 (LCN15), in the biological sample. The detection of the one or more marker proteins can be used to evaluate a renal cancer state, especially to detect renal cancer in the patient or provide an indication that the biological sample originates from a patient who may or may not be indicated of having renal cancer. The state of having or not having renal cancer or being or not being suspected of having renal cancer depends on the detection or failure to detect the one or more marker proteins. Likewise, the invention also provides the use of the one or more marker proteins selected from Haptoglobin (HP), Serum amyloid A1 (SAA1), and Lipocalin 15 (LCN15), in the diagnosis of renal cancer or in the evaluation of a renal cancer state. The evaluation of a renal cancer state is also referred to as evaluation for having renal cancer and it may comprise determining a likelihood of renal cancer, determining a suspicion of renal cancer or a risk of having renal cancer, diagnosing renal cancer, or the risk of developing renal cancer. All related to the patient from whom the biological sample originates. These references to renal cancer may refer to a specific type of renal cancer or distinguishing between types of renal cancer. Types of renal cancer are e.g. renal cell carcinoma (RCC), and RCC subtypes such as clear cell renal cell carcinoma (ccRCC), non-clear cell renal cell carcinoma (nccRCC), or nccRCC subtypes such as papillary renal cell carcinoma (pRCC) and chromophobe renal cell carcinoma (chRCC). In preferred embodiments, the renal cancer is ccRCC. In other embodiments the renal cancer is nccRCC. In particular preferred embodiments the invention distinguishes between ccRCC and nccRCC. nccRCC may be one of its subtypes: papillary, chromophobe, translocation, medullary, collecting duct, unclassified (none of the others listed before). Information on RCC types can be found in Naik et al., Ther Adv Urol 2024, 16: 1-17.

Preferably, the renal cancer is renal cell carcinoma (RCC). RCC examples are clear cell renal cell carcinoma (ccRCC) and non-clear cell renal cell carcinoma (nccRCC). In particular preferred embodiments the renal cancer is clear cell renal cell carcinoma (ccRCC). The invention provides protein markers which can be used in addition to, or as a replacement of, hematuria to reliably detect ccRCC. In other embodiments, the renal cancer may be nccRCC. The marker proteins, especially for diagnosing nccRCC, may be selected from H1-3 (H1.3 linker histone, cluster member), SLIT1 (Slit guidance ligand 1), STEAP2 (STEAP2 metal-loreductase), MATR3 (Matrin 3), or any combination thereof, especially preferred a combination with MATR3. Such a method would be a method of detecting renal cancer markers in a biological sample from a patient suspected of or to be evaluated for having renal cancer comprising detecting one or more marker proteins selected from H1-3, SLIT1, STEAP2 and MATR3, in the biological sample. All embodiments disclosed for the detection of marker proteins selected from Haptoglobin (HP), Serum amyloid A1 (SAA1), and Lipocalin 15 (LCN15), also apply to this method in preferred embodiments. H1-3, SLIT1, STEAP2 and MATR3 can also be used for the distinction between ccRCC and nccRCC. Higher amounts of H1-3, SLIT1, STEAP2 and/or MATR3 in the biological sample as compared to a healthy control sample indicate nccRCC. Higher amounts of HP, SAA1 and/or LCN15 in the biological sample as compared to a healthy control sample or to a nccRCC reference sample indicate ccRCC.

The biological sample can be obtained or has been obtained from a patient suspected of or to be evaluated for having renal cancer. The known health state of the patient immediately before or when performing the method of the invention may or may not be healthy. Preferably the patient is a mammal, especially preferred a human.

Preferably the biological sample comprises urine. Urine is an ideal source of biomarkers for renal cancer detection, especially of ccRCC, due to the low invasiveness, easy accessibility, and the kidney's role in filtering urine. The proximal convoluted tubule, where renal cancer like ccRCC arises, is responsible for filtering and reabsorbing solutes between blood and urine (Burg et al., American Journal of Physiology-Legacy Content, 1976, 231(2): 627-637) increasing the likelihood that secreted or shed biomarkers are present and detectable in the secreted urine fraction. Furthermore, renal cancer, especially ccRCC, predominantly affects people of the age of 60 and upwards, and very rarely younger individuals. In many countries, the older population is already part of population-based screening programs and health check-ups in which urine is routinely donated and analyzed, meaning that it might function as a biomarker pool for early detection of renal cancer.

The inventive marker proteins are also referred to as diagnostic biomarkers or just biomarkers herein. These are Serum amyloid A1 (SAA1), Haptoglobin (HP) and Lipocalin 15 (LCN15). They have been found in urine samples but can also be present in other body fluids. However, their presence in urine samples is most indicative of the renal cancer state. These markers can be used to diagnose renal cancer and are also suitable for the diagnosis of early-stage renal cancer. The markers can be detected individually or in combination. Preferred combinations are SAA1 and HP; or SAA1 and LCN15; or HP and LCN15; or SAA1 and HP and LCN15. Preferably the one or more marker proteins are Lipocalin 15, Serum amyloid A1, and Haptoglobin. A combination can mean that the marker proteins are detected individually and the detection data is combined to determine the renal cancer state. Individual detection can mean that one or more of the marker proteins are detected in a detection method together; the marker detection information being distinguishable between the marker proteins. E.g. parallel reaction monitoring mass spectrometry (PRM-MS) can be used for rapid and sensitive detection of SAA1, HP and LCN15. Combined detection data can be in the form of a score. An example is the combination of SAA1, HP and LCN15 detection into a UrineScore as explained in the examples. Such a combination had a performance accuracy of 96% in receiver operating characteristic curve (ROC) analysis for classification of ccRCC versus control cases.

The proteins with gene symbols or gene names like HP, SAA1 or LCN15 are known in the art and identified in the Human Protein Atlas (Uhlén et al., Science, 2015, 347(6220): 1260419; www.proteinatlas.org) or in the HUGO Gene Nomenclature Committee database (HGNC, genenames.org). Further gene symbols are used herein and their proteins are known in the art and identified by these databases.

Preferably detecting one or more marker proteins (or further proteins as control) comprises a determination of amounts to the one or more marker proteins or their protein fragments by mass spectrometry (MS). Mass spectrometry may be selected from electrospray ionization mass spectrometry (ESI-MS), ESI-MS/MS, ESI-MS/(MS)n (n is an integer greater than zero), matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SLMS), quadrupole time-of-flight (Q-TOF), atmospheric pressure chemical ionization mass spectrometry (APCI-MS), APCI-MS/MS, APCI-(MS)n, atmospheric pressure photoionization mass spectrometry (APPI-MS), APPI-MS/MS, and APPI-(MS)n. Other mass spectrometry methods may include, inter alia, quadrupole, fourier transform mass spectrometry (FTMS) and ion trap. In particular preferred are matrix-assisted laser desorption/ionization mass spectrometry (MALDI MS), surface-enhanced laser desorption/ionization mass spectrometry (SELDI MS), time of flight mass spectrometry (TOF MS) and liquid chromatography mass spectrometry (LC MS). LC MS is preferred. MS can be tandem mass spectrometry (MS/MS or with more than two MS stages). In particular preferred embodiments, MS is a selected reaction monitoring MS, such as parallel reaction monitoring mass spectrometry (Peterson et al., Molecular & Cellular Proteomics, 2012, 11(11): 1475-1488). In a selected reaction monitoring MS an ion of a particular mass is selected in the first stage of a tandem mass spectrometry and an ion product of a fragmentation reaction of the precursor ions is selected in the second mass spectrometry stage for detection. In particular preferred is parallel reaction monitoring mass spectrometry (PRM-MS), which allows parallel detection of many or all ions in a single analysis (Peterson et al., Molecular & cellular proteomics, 2012, 11(11): 1475-1488).

The determination of the presence and/or amount of the one or more marker proteins or their protein fragments, and hence a marker protein profile, may use one or more separation methods, such as liquid chromatography (LC), including high performance liquid chromatography (HPLC), in particular preferred Nano liquid chromatography (Nano-LC), which is a miniaturisation of HPLC. Further or alternative separation occurs during mass spectrometry, as mentioned above. Other suitable separation methods may include chemical extraction partitioning, column chromatography, ion exchange chromatography, hydrophobic (reverse phase) liquid chromatography, isoelectric focusing, one-dimensional polyacrylamide gel electrophoresis (PAGE), two-dimensional polyacrylamide gel electrophoresis (2D-PAGE) or other chromatography, such as thin-layer, gas or liquid chromatography, or any combination thereof. The chromatography, e.g. LC, max comprise a stationary phase. The stationary phase may be a porous material, such as porous silica or a porous polymer. An example is a monolith column.

In one embodiment, laser desorption/ionization time-of-flight mass spectrometry is used to determine the presence and/or amount of the one or more marker proteins or their protein fragments, and hence create a marker protein profile where the marker proteins are proteins or protein fragments that have been ionized and vaporized off an immobilizing support by incident laser radiation. A profile is then created by the characteristic time-of-flight for each protein, which depends on its mass-to-charge ("m/z") ratio.

The inventive method may comprise a separation method, such as liquid chromatography, followed by a mass spectroscopy, such as the combination LC MS. The mass spectroscopy may contain further separation of the marker proteins or their fragments. In particular preferred embodiments of the invention mass spectrometry is parallel reaction monitoring mass spectrometry.

Preferably detecting one or more marker proteins comprises fragmenting the one or more marker proteins to protein fragments. Protein fragments are also referred to as peptide fragments or peptides herein. The protein fragments can be detected, e.g. by mass spectrometry (MS) in a mass spectrometer detector. This, by way of their fragments, detects the marker proteins. Fragmenting can be done before and/or during mass spectrometry.

Preferably fragmenting is done to obtain protein fragments of a size of 4 to 50 amino acids, preferably of 5 to 30 amino acids, even more preferred of 6 to 20 amino acids. Such protein fragments may be selected for detection, e.g. by mass spectrometry. Preferably fragmenting the one or more marker proteins comprises enzymatic digestion. Enzymatic digestion can be proteolytic cleavage, e.g. with an endopeptidase. The endopeptidases may be one or more serine peptidases and/or one or more lysyl bond specific proteinases. Example endopeptidases are LysC endopeptidase (also referred to as lysyl endopeptidase, EC 3.4.21.50) and/or trypsin.

In particular preferred embodiments, the marker proteins fragments are for Lipocalin 15 (LCN15): selected from fragments with amino acid sequences DHLSMSTRA (SEQ ID NO: 1), VGSEGHFR (SEQ ID NO: 2), VPALGYLDVR (SEQ ID NO: 3), TQDVSPQALK (SEQ ID NO: 4); for Serum amyloid A1 (SAA1): selected from fragments with amino acid sequences GPGGVWAAEAISDAR (SEQ ID NO: 5), FFGHGAEDSLADQAANEWGR (SEQ ID NO: 6); for Haptoglobin (HP): selected from fragments with amino acid sequences NPANPVQR (SEQ ID NO: 7), GSFPWQAK (SEQ ID NO: 8), DIAPTLTLYVGK (SEQ ID NO: 9), VGYVSGWGR (SEQ ID NO: 10), HYEGSTVPEK (SEQ ID NO: 11), SCAVAEYGVYVK (SEQ ID NO: 12), VTSIQDWVQK (SEQ ID NO: 13); for Uromodulin (UROM): selected from fragments with amino acid sequences FVGQGGAR (SEQ ID NO: 14), DWVSWTPAR (SEQ ID NO: 15), DGPCGTVLTR (SEQ ID NO: 16), INFACSYPLDMK (SEQ ID NO: 17), SGSVIDQSR (SEQ ID NO: 18), VLNLGPITR (SEQ ID NO: 19); for Kallikrein-1 (KLK1): selected from fragments with amino acid sequences QWVLTAAHCISDNYQLWLGR (SEQ ID NO: 20), QADEDYSHDLMLLR (SEQ ID NO: 21), LTEPADTITDAVK (SEQ ID NO: 22), ILPNDECK (SEQ ID NO: 23); for Apolipoprotein D (APOD): selected from fragments with amino acid sequences CPNPPVQENFDVNK (SEQ ID NO: 24), IPTTFENGR (SEQ ID NO: 25), NILTSNNIDVK (SEQ ID NO: 26), MTVTDQVNCPK (SEQ ID NO: 27). The fragments may comprise or consist of these amino acid sequences. The inventive method may detect one or more of these fragments for Lipocalin 15, Serum amyloid A1, and/or Haptoglobin (preferably Lipocalin 15, Serum amyloid A1, and Haptoglobin), preferably at least two of these fragments for Lipocalin 15, Serum amyloid A1, and/or Haptoglobin are detected. In the embodiments detecting the further proteins (control proteins) the inventive method may detect one or more of these fragments for Uromodulin, Kallikrein-1, and/or Apolipoprotein D, preferably at least two of these fragments for Uromodulin, Kallikrein-1, and/or Apolipoprotein D are detected.

In preferred embodiments only a few marker proteins are analysed in the inventive method, such as at most 100, or at most 50, or at most 10, or even only Lipocalin 15, Serum amyloid A1, and Haptoglobin. This number of marker proteins may be independent of the further proteins (control proteins). In other embodiments the total number of proteins analysed (marker proteins and further proteins) is at most 100 or at most 50 or at most 10, or even only Lipocalin 15, Serum amyloid A1, Haptoglobin, Uromodulin, Kallikrein-1, and Apolipoprotein D.

Mass spectrometry may further comprise ionization and/or fragmentation. Such a fragmentation may happen during ionization. If an enzymatic digestion is done then the fragmentation during mass spectrometry is a further fragmentation, after the enzymatic digestion. Preferably detecting protein fragments by mass spectrometry comprises the determination of an amount of the protein fragments, e.g. protein fragments from proteins that have been enzymatically digested and/or fragmented during MS. Fragmentation occurs similarly for marker proteins and control proteins.

Determination of amounts to the one or more marker proteins preferably comprises determining a total signal intensity detected by mass spectrometry associated with any one of the one or more marker proteins. Signal intensity associated with a particular marker protein includes signals of its fragments that can be associated with the particular marker protein.

A further method variation comprises detecting one or more marker proteins by affinity binding. An example of affinity binding comprises binding the marker proteins or their fragments with an affinity binding partner, such as an antibody or aptamer. The affinity binding partner shall bind the marker proteins of their fragments specifically and/or with high affinity of e.g. $10^6$ L/mol or higher, preferably $10^7$ L/mol or higher, or even more preferred $10^8$ L/mol or higher. Antibody affinity is determined by comparing the rate of the affinity complex formation to the rate of dissociation. Binding can be detected by a detectable marker, preferably a detectable marker linked to the affinity binding partner. Such detectable markers can e.g. be radioactive labels, quantum dot labels, enzyme labels, fluorescent labels, or colour labels. A preferred example of an affinity binding method is ELISA (enzyme-linked immunosorbent assay), which uses an enzyme as label. The enzyme label can catalyse a reaction that creates a detectable signal. Detection by the affinity binding can be quantized. Preferably the method comprises determining amounts of the one or more marker proteins by affinity binding.

For making an association with a renal cancer state, e.g. for evaluating if a patient has renal cancer, a comparison is usually done with a healthy state control (healthy control) and/or a comparable diseased state reference (disease reference). Preferably an amount of the one or more marker proteins in the biological sample is determined and compared to the amount of the one or more marker proteins in a healthy control sample and/or compared to the amount of the one or more marker proteins in a disease reference sample. Such a comparison can be used to determine if the patient has or has not renal cancer, e.g. by determining if the amounts closely resemble (or not) a healthy control and/or a disease reference.

It is also possible to make a comparison with further proteins that are not indicative of renal cancer. These further proteins can be used as control proteins. In preferred embodiments one or more further proteins that are not indicative of renal cancer are determined as control proteins in the biological sample. Preferably the further proteins are renal housekeeping proteins. Housekeeping proteins are usually independent of a renal cancer state. Preferably the housekeeping protein is a protein which is (i) found in healthy control samples, nccRCC samples and ccRCC samples, and (ii) had expression values which did not differ by more than one order of magnitude for each individual housekeeping protein across all three groups selected from healthy control samples, nccRCC samples and ccRCC samples. Housekeeping proteins are e.g. Uromodulin (UMOD), Kallikrein-1 (KLK1) Apolipoprotein D (APOD). Preferably the further proteins that are not indicative of renal cancer are selected from Uromodulin, Kallikrein-1, and Apolipoprotein D. Preferably the inventive method comprises determining the amount of the control proteins (i.e. the further proteins that are not indicative of renal cancer) in the biological sample. The relation of the amount of the one or more marker proteins in the biological sample to the amount of the one or more further proteins in the biological sample can be determined. This relation can be used as an indicator if the marker proteins are present in elevated amounts. The control proteins can be used as reference for normal amounts, especially when the relation in the amounts with the marker proteins is known for healthy controls and/or for disease references. Any treatment and detection, e.g. fragmentation and detection by MS, as described for marker proteins shall also apply for the control proteins. Preferably, the control proteins from the samples are treated together and/or identically as the marker proteins.

Several steps of the inventive method embodiments can be combined. E.g. preferably the biological sample is a urine sample, the method further comprising precipitating a protein fraction of the urine sample, removing a supernatant from the protein fraction, resuspending the protein fraction, fragmenting the one or more marker proteins to protein fragments, chromatographically separating protein fragments, detecting protein fragments by mass spectrometry.

Preferably the inventive method further comprises determining the amount of protein fragments of one or more further proteins that are not indicative of renal cancer (which are determined as control proteins) in the biological sample. Preferably the further proteins are renal housekeeping proteins. Especially preferred the further proteins that are not indicative of renal cancer are selected from Uromodulin, Kallikrein-1, and Apolipoprotein D. As described above, the inventive method may further comprise determining if a patient has renal cancer or is suspected of having renal cancer by comparing the amount of the protein fragments of the one or more marker proteins to the amount of the one or more further proteins. Given that the marker proteins (or their amounts) are indicative of the renal cancer type or subtype (e.g. ccRCC or nccRCC or nccRCC subtypes, in particular the renal cancer state), the inventive method can be used to determine the type (or subtype) of renal cancer that a patient has, by comparing the amount of the protein fragments of the one or more marker proteins to the amount of the one or more further proteins.

In particular preferred embodiments, the renal cancer state (e.g. renal cancer diagnosed, ccRCC diagnosed, nccRCC diagnosed, or ccRCC distinguished over nccRCC) is evaluated by determining the amount of the protein fragments of a marker protein in relation to the amount of the protein fragments of one or more renal housekeeping proteins. This can be repeated for each of the one or more marker proteins. This evaluation via this relation can be compared between the biological sample from the patient to be evaluated in comparison to a healthy control sample. In the biological sample and the healthy control sample the same marker proteins and the same housekeeping proteins are determined. An increase in this relation of the marker protein in the biological sample from the patient in comparison to the marker protein in the healthy control sample can indicate the renal cancer state. Preferably the amount relation for all marker proteins selected from Haptoglobin (HP), Serum amyloid A1 (SAA1), and Lipocalin 15 (LCN15) is determined. An increase of only one of these marker proteins may indicate a likelihood for nccRCC. An increase of two or all three marker proteins may indicate a likelihood for ccRCC.

If renal cancer is detected, indicated or a likelihood of renal cancer established by the present invention, then further methods may follow, such as a further renal cancer detection method, e.g. by a tomography or a biopsy, monitoring of the renal cancer, e.g. by one of the further renal cancer detection methods or by repeating the detection method of the present invention, or treatment methods, such as radiotherapy, chemotherapy or tumor resection.

A chemotherapy may include the administration of a therapeutically effective amount of an anti-cancer agent to treat the renal cancer. In some embodiments, a patient can be monitored for renal cancer using the methods and biomarkers as disclosed herein.

In general, a therapy is considered to "treat" renal cancer if it provides one or more of the following treatment outcomes: reduce or delay recurrence of the RCC after the therapy; increase median survival time or decrease metastases. In some embodiments, an anti-cancer therapy is, for example but not limited to administration of a chemotherapeutic agent, radiotherapy etc. The term "anti-cancer agent" or "anti-cancer drug" is any agent, compound or entity that would be capable of negatively affecting the cancer in the patient, for example killing cancer cells, inducing apoptosis in cancer cells, reducing the growth rate of cancer cells, reducing the number of metastatic cells, reducing tumor size, inhibiting tumor growth, reducing blood supply to a tumor or cancer cells, promoting an immune response against cancer cells or a tumor, preventing or inhibiting the progression of cancer, or increasing the lifespan of the patient with cancer. Anti-cancer agents include biological agents (biotherapy), chemotherapy agents, and radiotherapy agents. In some embodiments, the anti-cancer therapy includes a chemotherapeutic regimen and further comprises radiation therapy. In some embodiments, the anti-cancer treatment comprises the administration of a chemotherapeutic drug, alone or in combination with surgical resection of the tumor. In some embodiments, the treatment compresses radiation therapy and/or surgical resection of the tumor masses.

In some embodiments, a chemotherapeutic agent can be in the form of a prodrug which can be activated to a cytotoxic form. For example, chemotherapeutic drugs include, but are not limited to: temozolomide (Temodar), procarbazine (Matulane), and lomustine (CCNU). Chemotherapy given intravenously (by IV, via needle inserted into a vein) includes vincristine (Oncovin or Vincasar PFS), cisplatin (Platinol), carmustine (BCNU, BiCNU), and carboplatin (Paraplatin), Mexotrexate (Rheumatrex or Trexall), irinotecan (CPT-11); erlotinib; oxalipatin; anthracyclinsidarubicin and daunorubicin; doxorubicin; alkylating agents such as melphalan and chlorambucil; cis-platinum, methotrexate, and alkaloids such as vindesine and vinblastine. In some embodiments, the patients are administered with anti-VEGF agents. As used herein the term "anti-VEGF agent" refers to any compound or agent that produces a direct effect on the signaling pathways that promote growth, proliferation and survival of a cell by inhibiting the function of the VEGF protein, including inhibiting the function of VEGF receptor proteins. The term "agent" or "compound" as used herein means any organic or inorganic molecule, including modified and unmodified nucleic acids such as antisense nucleic acids, RNAi agents such as siRNA or shRNA, peptides, peptidomimetics, receptors, ligands, and antibodies. Preferred VEGF inhibitors, include an anti-VEGF antibody, preferably an anti-VEGF monoclonal antibody, such as AVASTIN® (bevacizumab). Additional VEGF inhibitors include CP-547,632 (3-(4-Bromo-2,6-difluoro-benzyloxy)-5-[3-(4-pyrrolidin 1-yl-butyl)-ureido]-isothiazole-4-carboxylic acid amide hydrochloride; Pfizer Inc., NY), AG13736, AG28262 (Pfizer Inc.), SU5416, SU1 1248, & SU6668 (formerly Sugen Inc., now Pfizer, New York, N.Y.), ZD-6474 (AstraZeneca), ZD4190 which inhibits VEGF-R2 and -R1 (AstraZeneca), CEP-7055 (Cephalon Inc., Frazer, Pa.), PKC 412 (Novartis), AEE788 (Novartis), AZD-2171), NEXAVAR® (BAY 43-9006, sorafenib; Bayer Pharmaceuticals and Onyx Pharmaceuticals), vatalanib (also known as PTK-787, ZK-222584: Novartis & Schering: AG), MACUGEN® (pegaptanib octasodium, NX-1838, EYE-001, Pfizer Inc./Gilead/Eyetech), IM862 (glufanide disodium, Cytran Inc. of Kirkland, Wash., USA), VEGFR2-selective monoclonal antibody DC101 (ImClone Systems, Inc.), angiozyme, a synthetic ribozyme from Ribozyme (Boulder, Colo.) and Chiron (Emeryville, Calif.), Sirna-027 (a siRNA-based VEGFRI inhibitor, Sirna Therapeutics, San Francisco, Calif.) Caplostatin, soluble ectodomains of the VEGF receptors, Neovastat (AEtema Zentaris Inc; Quebec City, Calif.) and combinations thereof.

The pharmaceutically "effective amount" for purposes herein is determined to be effective to achieve improvement including, but not limited to, improved survival rate or more rapid recovery, or improvement or elimination of symptoms and other indicators as are selected as appropriate measures by those skilled in the art.

Furthermore, it was also found that the urine of ccRCC patients indicated dysregulated metabolism throughout the urogenital tract with increased lipid metabolism and mitochondrial respiration in the patients. A therapy following the inventive renal cancer detection may ameliorate such an abnormal lipid metabolism and/or mitochondrial respiration.

The present invention further provides a kit comprising marker proteins or marker protein fragments, wherein the marker proteins are Lipocalin 15, Serum amyloid A1, and Haptoglobin; and wherein the marker protein fragments comprise fragments from each of marker proteins Lipocalin 15, Serum amyloid A1, and Haptoglobin.

The fragments of Lipocalin 15 (LCN15) are selected from fragments with amino acid sequences DHLSMSTRA (SEQ ID NO: 1), VGSEGHFR (SEQ ID NO: 2), VPALGYLDVR (SEQ ID NO: 3), TQDVSPQALK (SEQ ID NO: 4). The fragments of Serum amyloid A1 (SAA1) are selected from fragments with amino acid sequences GPGGVWAAEAISDAR (SEQ ID NO: 5), FFGHGAEDSLADQAANEWGR (SEQ ID NO: 6). The fragments of Haptoglobin (HP) are selected from fragments with amino acid sequences NPANPVQR (SEQ ID NO: 7), GSFPWQAK (SEQ ID NO: 8), DIAPTLTLYVGK (SEQ ID NO: 9), VGYVSGWGR (SEQ ID NO: 10), HYEGSTVPEK (SEQ ID NO: 11), SCAVAEYGVYVK (SEQ ID NO: 12), VTSIQDWVQK (SEQ ID NO: 13). The kit may optionally further comprise one or more further proteins or further protein fragments, wherein the further proteins are selected from Uromodulin, Kallikrein-1, and Apolipoprotein D; and wherein the further protein fragments comprise fragments from one or more of the further proteins Uromodulin, Kallikrein-1, and Apolipoprotein D. The fragments of Uromodulin (UROM) are selected from fragments with amino acid sequences FVGQGGAR (SEQ ID NO: 14), DWVSWTPAR (SEQ ID NO: 15), DGPCGTVLTR (SEQ ID NO: 16), INFACSYPLDMK (SEQ ID NO: 17), SGSVIDQSR (SEQ ID NO: 18), VLNLGPITR (SEQ ID NO: 19). The fragments of Kallikrein-1 (KLK1) are selected from fragments with amino acid sequences QWVLTAAHCISDNYQLWLGR (SEQ ID NO: 20), QADEDYSHDLMLLR (SEQ ID NO: 21), LTEPADTITDAVK (SEQ ID NO: 22), ILPNDECK (SEQ ID NO: 23). The fragments of Apolipoprotein D (APOD) are selected from fragments with amino acid sequences CPNPPVQENFDVNK (SEQ ID NO: 24), IPTTFENGR (SEQ ID NO: 25), NILTSNNIDVK (SEQ ID NO: 26), MTVTDQVNCPK (SEQ ID NO: 27). The fragments may comprise or consist of these amino acid sequences.

In preferred embodiments, the kit is a small kit with only a few components. In preferred embodiments only a few marker proteins (or their fragments) are contained in the kit, such as at most 100 or at most 50 or at most 10, or even only Lipocalin 15, Serum amyloid A1, and Haptoglobin. This number of marker proteins may be independent of the further proteins (control proteins). In other embodiments the total number (of marker proteins and further proteins together) of proteins (or their fragments) is at most 100, or at most 50, or at most 10, or even only Lipocalin 15, Serum amyloid A1, Haptoglobin, Uromodulin, Kallikrein-1, and Apolipoprotein D (or their fragments).

The kit (in particular the proteins or fragments therein) can be used as test standards for the inventive method. E.g. the proteins or fragments can be used to detect a diagnostic peptide signature of the marker proteins for calibrating a detection device, such as a MS device, before sample analysis. E.g. the kit can be used to determine the chromatographical elution time and/or peak pattern of the proteins or their fragments.

The marker proteins, further proteins or their fragments can be provided in containers such as vials. Preferably, the marker proteins, further proteins or their fragments are in solution or in solid, in particular freeze-dried, state. They are preferably not attached to other moieties, such as a chip or other immobilization moieties. The solid marker proteins, further proteins or their fragments should be solvable, i.e. able to be dissolved by putting them in an aqueous liquid, preferably water.

The kit may further comprise one or more endopeptidases, such as LysC (Lysyl Endopeptidase®) and/or trypsin.

The kit may further comprise one or more fluids for a chromatograph, such as water, acetone, acetonitrile, and/or formic acid. The fluids can be provided in containers, such as vials.

The kit may further comprise instructions for performing the inventive method.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. $\pm 10\%$.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range and "comprising" relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The present invention is further illustrated by the following figures and examples, without being limited to these embodiments of the invention.

## Figures

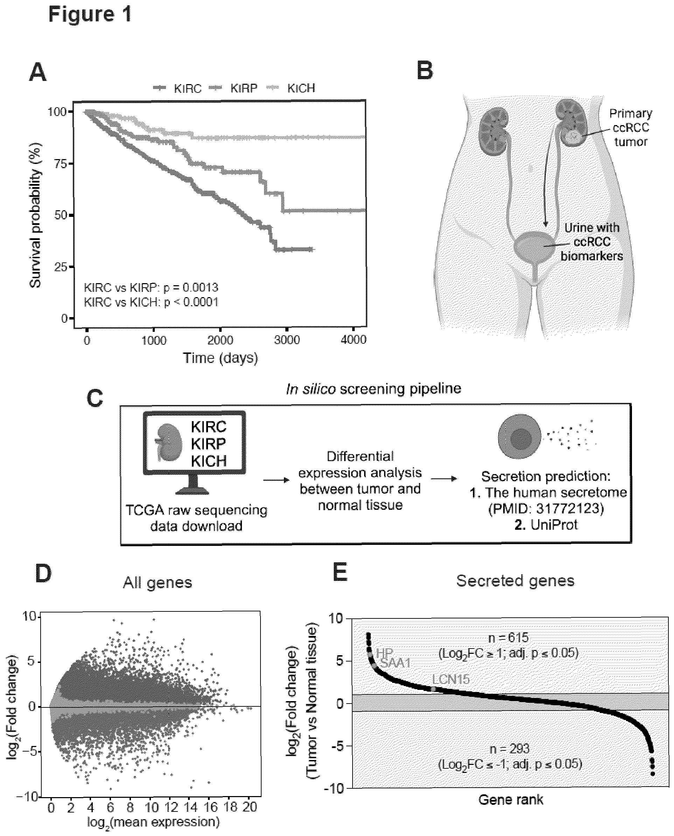

### Figure 1. Analysis of TCGA data reveals potential secreted biomarkers for ccRCC

**A.** Survival analysis of the three different kidney cancer types present in The Cancer Genome Atlas (TCGA): Clear cell renal cell carcinoma (KIRC), papillary renal cell carcinoma (KIRP) and chromophobe renal cell carcinoma (KICH). Statistical significance between different cancer types assessed with pairwise Logrank tests.

**B.** Schematic of enriched and secreted proteins in ccRCC patients passing through the urogenital tract and accumulating in the urine bladder before discharge. Schematic created with BioRender.com.

**C.** Schematic of in silico screen performed to find secreted biomarker candidates for ccRCC. Differential expression analysis between tumors and normal tissue in KIRC, KIRP and KICH was performed and followed by filtering for genes with a secretion signal judged by presence of a signaling peptide and absence of transmembrane regions. Schematic created with BioRender.com

**D.** Differential expression analysis of all genes between KIRC tumor and KIRC normal tissue. Genes highlighted in red are upregulated in tumors compared to normal tissue, whereas genes highlighted in blue are downregulated.

**E.** Gene rank plot showing upregulated (red) and downregulated (blue) genes between KIRC tumor and KIRC normal tissue. An upregulated gene is defined as having a log2 FC $\geq$ 1 and adjusted p value of $\leq$ 0.05 and a downregulated gene is defined as having a log2 FC < -1 and adjusted p value of $\geq$ 0.05. Adjusted p-values calculated through Benjamini-Hochberg adjusted Wald tests.

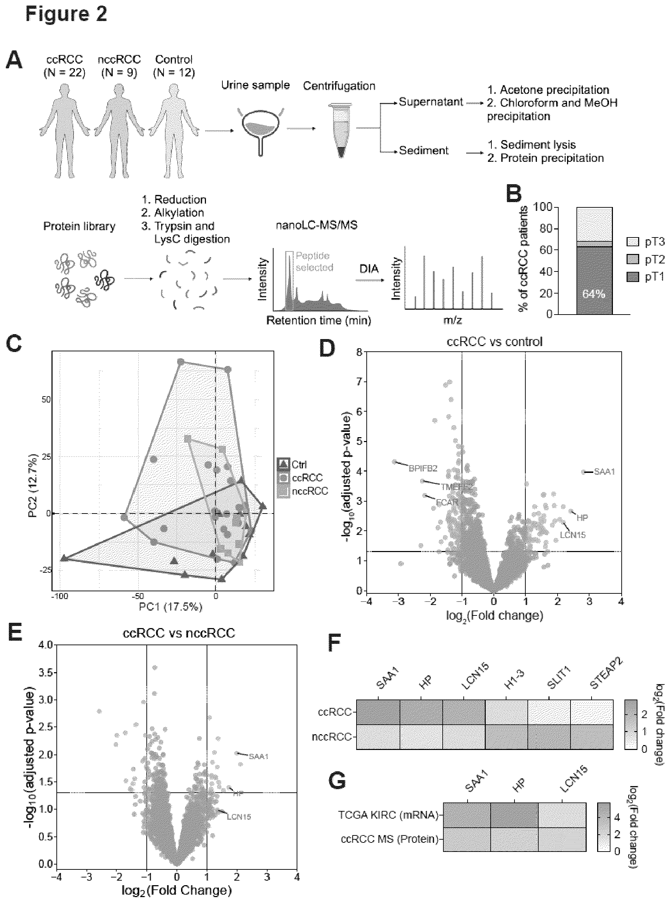

### Figure 2. Urine supernatant from a renal cell carcinoma patient cohort indicates diagnostic biomarkers for ccRCC

**A.** Schematic of workflow for diagnostic biomarkers discovery from renal cell carcinoma patients using proteomics. Abbreviations: ccRCC = clear cell renal cell carcinoma, nccRCC = non-clear cell renal cell carcinoma, MeOH = Methanol, LC = Liquid chromatography, MS = Mass spectrometry, DIA = data-independent acquisition. Schematic created using BioRender.com.

**B.** Distribution of tumor stages at the timepoint of presentation in the clinic for ccRCC patients. Abbreviations: pT1 = Tumor stage 1. The tumor is a maximum of 7 cm across. pT2 = Tumor stage 2. The tumor is larger than 7 cm across. pT3 = Tumor stage 3. The tumor has grown into a major renal vein (e.g. vena cava or renal vein) or into neighboring tissues but has not spread past Gerota's fascia or into the adrenal gland.

**C.** Principal component analysis plot on mass spectrometry data from control, ccRCC and nccRCC patient groups.

**D.** Volcano plot showing upregulated (red) and downregulated (blue) proteins in ccRCC patients compared to controls. Adjusted p values calculated via limma-moderated Benjamini-Hochberg-corrected two-sided t-test. FC = fold change.

**E.** Volcano plot showing upregulated (red) and downregulated (blue) proteins in ccRCC patients compared to nccRCC patients. Adjusted p values calculated via limma-moderated Benjamini-Hochberg-corrected two-sided t-test. FC = fold change.

**G.** Heatmap showing fold change between ccRCC patient urine and control urine, and nccRCC patient urine and control urine for the top three upregulated proteins in ccRCC and nccRCC patients. FC = Fold change.

**F.** Heatmap showing fold change between tumor and healthy surrounding tissue (KIRC, TCGA RNA sequencing data), and between ccRCC patient urine and control urine (proteomics data) for SAA1, HP and LCN15. Abbreviations: TCGA = The cancer genome atlas, MS = Mass spectrometry, FC = Fold change.

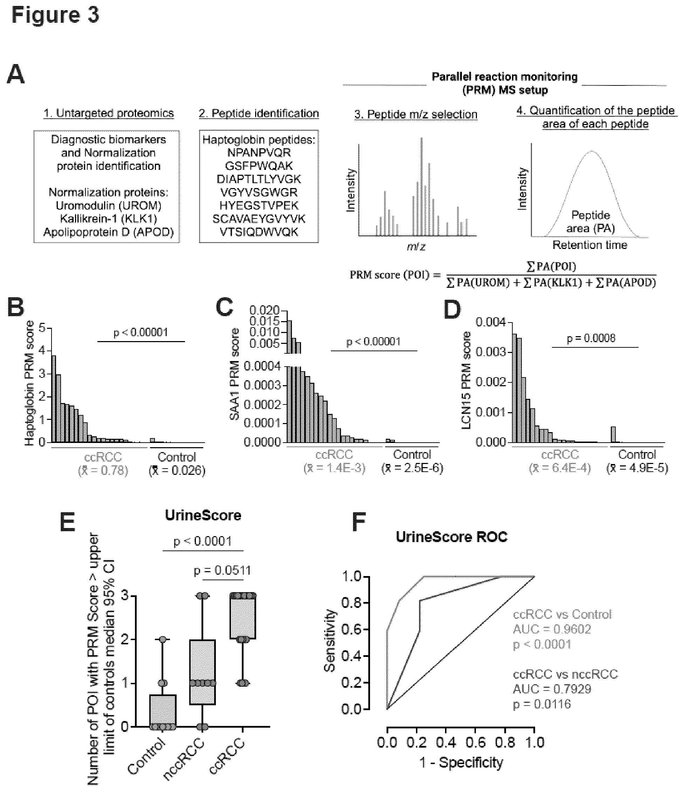

**Figure 3. Parallel reaction monitoring mass spectrometry accurately diagnoses ccRCC**

**A.** Schematic of the PRM-MS method used to quantify the SAA1, HP and LCN15 levels in urine samples. Levels are determined based on normalization to three housekeeping proteins (UMOD, KLK1 and APOD). The peptide area for each detected peptide from a specified protein is quantified, detected peptides from Haptoglobin are shown as an example. Abbreviations: PA = Peptide area, POI = Protein of interest (SAA1, HP or LCN15). Schematic created using BioRender.com. Shown Haptoglobin peptides are NPANPVQR (SEQ ID NO: 7), GSFPWQAK (SEQ ID NO: 8), DIAPTLTLYVGK (SEQ ID NO: 9), VGYVSGWGR (SEQ ID NO: 10), HYEGSTVPEK (SEQ ID NO: 11), SCAVAEYGVYVK (SEQ ID NO: 12), VTSIQDWVQK (SEQ ID NO: 13).

**B.** Waterfall plot of PRM score for Haptoglobin compared between ccRCC and Control cohorts. Statistical significance assessed through Mann-Whitney U-test. $\bar{x}$ = sample mean.

**C.** Waterfall plot of PRM score for SAA1 compared between ccRCC and Control cohorts. Statistical significance assessed through Mann-Whitney U-test. $\bar{x}$ = sample mean.

**B.** Waterfall plot of PRM score for LCN15 compared between ccRCC and Control cohorts. Statistical significance assessed through Mann-Whitney U-test. $\bar{x}$ = sample mean.

**D.** Box plot of UrineScore for controls, nccRCC and ccRCC patients. Per protein of interest (POI) per patient or control, a value of 1 is derived if the PRM score is higher than the upper limit of the 95% CI for the control. Statistical significance assessed through Kruskal-Wallis test with Dunn's multiple comparisons test.

**E.** Receiver operating characteristic curve to assess the performance of the UrineScore in differentiating between ccRCC and control samples, and between ccRCC and nccRCC samples. Performance quantified through Area under the receiver operating characteristic curve (AUC) analysis.

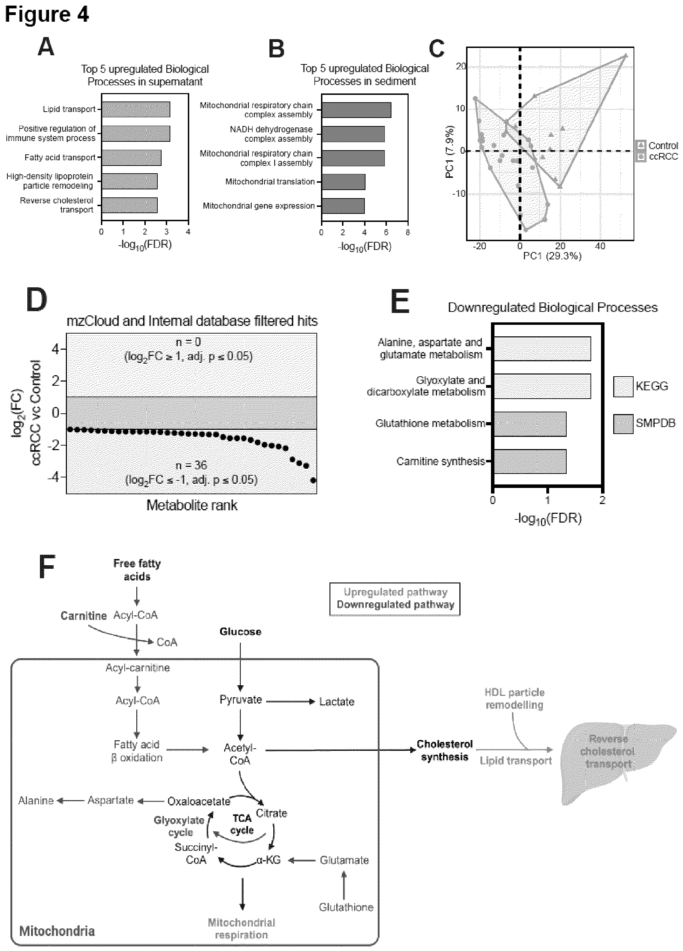

**Figure 4. ccRCC patient urine indicates metabolic dysregulation with increased mitochondrial respiration and lipid metabolism**

**A.** Gene ontology (GO) term analysis using Enrichr on all upregulated proteins in ccRCC urine supernatant compared to Control urine supernatant. FDR = False discovery rate.

**B.** GO term analysis using Enrichr on all upregulated proteins in ccRCC urine sediment compared to Control urine sediment. FDR = False discovery rate.

**C.** Principal component analysis plot on metabolomics data from Control and ccRCC patient groups.

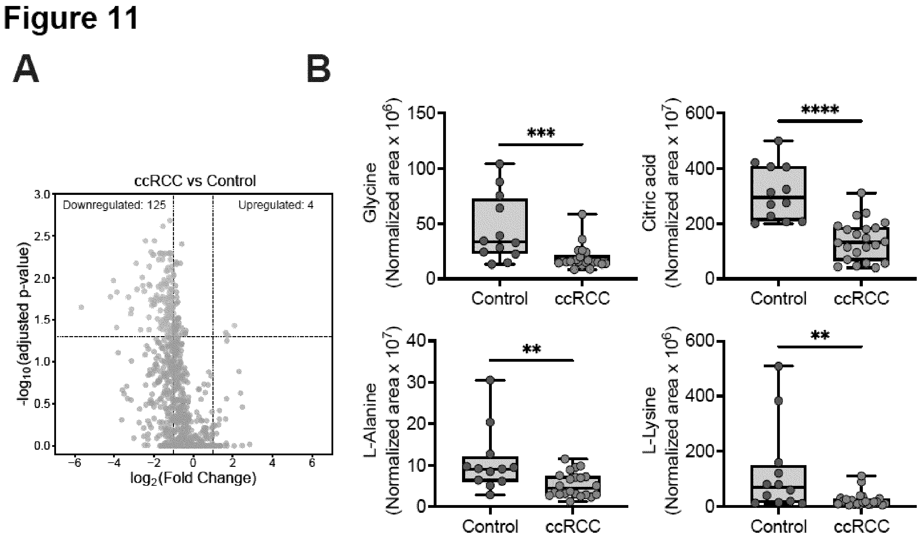

**D.** Metabolite rank plot showing upregulated (red) and downregulated (blue) metabolites found in the mzCloud and an internal database in ccRCC urine supernatant compared to controls. An upregulated metabolite is defined as having a log2 FC $\geq$ 1 and adjusted p value of $\leq$ 0.05 and a downregulated metabolite is defined as having a log2 FC $\leq$ -1 and adjusted p value of $\geq$ 0.05. FC = Fold change. Significance assessed via limma-moderated Benjamini-Hochberg-corrected two-sided t-test. Full, unfiltered, dataset is presented in Figure 11.

**F.** GO term analysis using Metaboanalyst on all downregulated metabolites in ccRCC urine supernatant compared to Control urine supernatant. The downregulated metabolites were matched against two different databases: KEGG and SMPDB. FDR = False discovery rate.

**G.** Schematic of proposed altered metabolic network in the urogenital tract of ccRCC patients. Green represents upregulated pathways and red represents downregulated pathways. CoA = Coenzyme A, $\alpha$-KG = alpha-ketoglutarate.

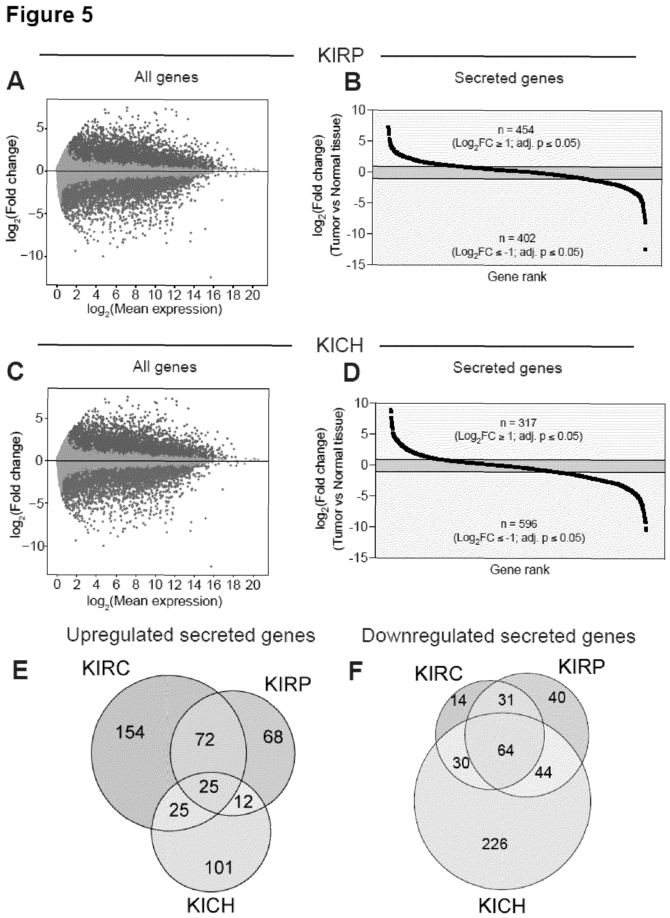

**Figure 5. KIRP and KICH have unique secretory profiles**

**A.** Differential expression analysis of all genes between KIRP tumor and KIRP normal tissue. Genes highlighted in red are upregulated in tumors compared to normal tissue, whereas genes highlighted in blue are downregulated.

**B.** Gene rank plot showing upregulated (red) and downregulated (blue) genes between KIRP tumor and KIRP normal tissue. An upregulated gene is defined as having a log2 Fold change $\geq$ 1 and adjusted p value of $\leq$ 0.05 and a downregulated gene is defined as having a log2 Fold change $\leq$ -1 and adjusted p value of $\geq$ 0.05. Adjusted p-values calculated through Benjamini-Hochberg adjusted Wald tests.

**C.** Differential expression analysis of all genes between KICH tumor and KICH normal tissue. Genes highlighted in red are upregulated in tumors compared to normal tissue, whereas genes highlighted in blue are downregulated.

**D.** Gene rank plot showing upregulated (red) and downregulated (blue) genes between KICH tumor and KICH normal tissue. An upregulated gene is defined as having a log2 FC $\geq$ 1 and adjusted p value of $\leq$ 0.05 and a downregulated gene is defined as having a log2 FC < -1 and adjusted p value of $\geq$ 0.05. Adjusted p-values calculated through Benjamini-Hochberg adjusted Wald tests.

**E.** Venn diagram showing the overlap of all upregulated, secreted genes for KIRC, KIRP and KICH

**F.** Venn diagram showing the overlap of all downregulated, secreted genes for KIRC, KIRP and KICH.

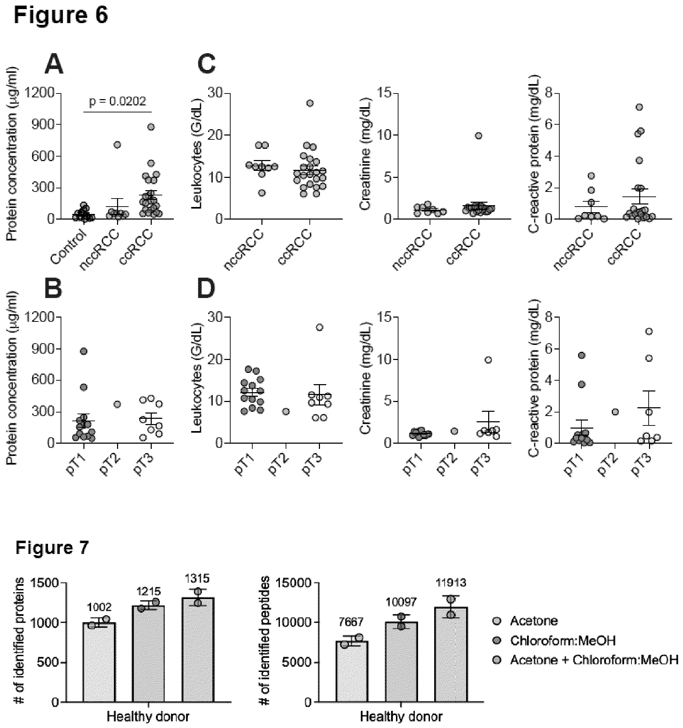

**Figure 6. Clinical cohort overview data**

**A.** Protein concentration in urine supernatant from control, nccRCC patients and ccRCC patients determined via Bradford protein assay. * = p < 0.005

**B.** Leukocyte, Creatinine and C-reactive protein levels in serum, respectively, for nccRCC patients and ccRCC patients

**A.** Protein concentration in urine supernatant from ccRCC patients divided into pT1, pT2 and pT3 stages determined via Bradford protein assay. Abbreviations: pT1 = Tumor stage 1. The tumor is a maximum of 7 cm across. pT2 = Tumor stage 2. The tumor is larger than 7 cm across. pT3 = Tumor stage 3. The tumor has grown into a major renal vein (e.g. vena cava or renal vein) or into neighboring tissues but has not spread past Gerota's fascia or into the adrenal gland.

**B.** Leukocyte, Creatinine and C-reactive protein levels in serum, respectively, ccRCC patients divided into pT1, pT2 and pT3 stages. Abbreviations: pT1 = Tumor stage 1. The tumor is a maximum of 7 cm across. pT2 = Tumor stage 2. The tumor is larger than 7 cm across. pT3 = Tumor stage 3. The tumor has grown into a major renal vein (e.g. vena cava or renal vein) or into neighboring tissues but has not spread past Gerota's fascia or into the adrenal gland.

**Figure 7. Double protein precipitation increases unique peptide and protein yield from urine supernatant**
Number of identified unique proteins (left) and unique peptides (right) in a healthy control urine supernatant sample using three different protein precipitation methods. (i) Precipitation only with acetone, (ii) precipitation only with chloroform and methanol, and (iii) double protein precipitation with acetone followed by chloroform and methanol. Two technical replicates per sample is shown. MeOH = Methanol.

**Figure 8. Urine supernatant in nccRCC patients reveal upregulated and downregulated proteins compared to controls**
Volcano plot showing upregulated (red) and downregulated (blue) proteins in nccRCC patients compared to controls. Adjusted p values calculated via limma-moderated Benjamini-Hochberg-corrected two-sided t-test. FC = fold change.

**Figure 9. Urine sediment proteomics reveals metabolic dysregulation in ccRCC patients**

**A.** Principal component analysis plot on mass spectrometry data from control, ccRCC and nccRCC patient groups.

**B.** Volcano plot showing upregulated (red) and downregulated (blue) proteins in ccRCC patients compared to controls. Adjusted p values calculated via limma-moderated Benjamini-Hochberg-corrected two-sided t-test. FC = fold change.

**C.** Volcano plot showing upregulated (red) and downregulated (blue) proteins in nccRCC patients compared to controls. Adjusted p values calculated via limma-moderated Benjamini-Hochberg-corrected two-sided t-test. FC = fold change.

**D.** Volcano plot showing upregulated (red) and downregulated (blue) proteins in ccRCC patients compared to nccRCC patients. Adjusted p values calculated via limma-moderated Benjamini-Hochberg-corrected two-sided t-test. FC = fold change.

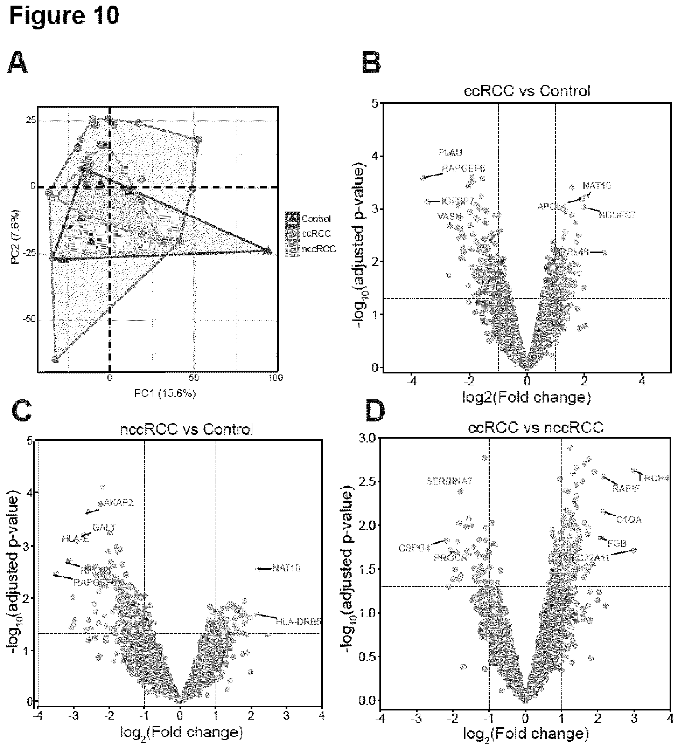

**Figure 10. PRM-MS enables detection of SAA1, HP and LCN15**

**A.** Heatmap showing mean normalized peak area per group for the three housekeeping proteins used in the PRM-MS.

**B.** Correlation matrix of SAA1, HP and LCN15 for combined control and ccRCC PRM scores. Correlation determined using Pearson's r.

**C.** Proportion of control and ccRCC samples in which SAA1, HP and LCN15, respectively, were detected with PRM-MS and PRM scores could be calculated.

**D.** Receiver operating characteristic curve to assess the performance of the PRM score of Haptoglobin, SAA1 and LCN15, respectively, in differentiating between control and ccRCC samples. Performance quantified through Area under the receiver operating characteristic curve (AUC) analysis.

### Figure 11. ccRCC urine indicate metabolite reduction compared to controls

**A.** Volcano plot showing upregulated (red) and downregulated (blue) metabolites in ccRCC patients compared to controls. Adjusted p values calculated via limma-moderated Benjamini-Hochberg-corrected two-sided t-test. FC = fold change.

**B.** Boxplots of 4 representative metabolites expression values (Glycine, Citric acid, L-Alanine and L-Lysine) as determined by metabolomics. ** = p < 0.01, *** = p < 0.001, **** = p < 0.0001.

## Examples

### Example 1: Materials and Methods

#### 1.1 TCGA kidney cancer data analysis

TCGA RNA sequencing data from tumor and normal tissue for TCGA-KIRC, TCGA-KIRP and TCGA-KICH was downloaded through the GDC portal. Differential expression analysis between tumor and matched normal tissue was performed with DESeq2 (RRID:SCR_015687, v1.22.2). Significantly upregulated or downregulated genes were defined as genes with ≥10 counts, and Benjamini-Hochberg adjusted Wald p values of $p < 0.05$. For secretion prediction, the HPA secretome classification was used, in which secreted proteins are defined as having a signaling peptide and no transmembrane domain, resulting in a list of 2641 secreted transcripts (Uhlén et al., Science signaling, 2019, 12(609): eaaz0274).

#### 1.2 Patient cohort

Urine was collected from 40 patients who presented with a suspected foreign primary renal mass at the urology department of the Medical University of Vienna. Out of the 40 patients, 9 were excluded due to the renal mass being identified as something other than a renal cell carcinoma, such as oncocytomas, cysts, angiomyolipoma, papillary adenoma or the kidney tumor was a metastasis. From the remaining 31 patients, 22 patients were characterized as ccRCC, 8 as pRCC and 1 as chromophobe RCC through histological assessment by a trained pathologist. For further analysis, the pRCC and chromophobe RCC patients were combined into one group of non-clear cell renal cell carcinoma (nccRCC) patients. Leukocytes, Creatinine and CRP levels were obtained in the routine blood analysis using standard methods at the Institute of Laboratory Medicine, Vienna General Hospital.

#### 1.3 Protein precipitation

3 ml of ccRCC patient, nccRCC patient or control urine was centrifuged at 1000x $g$ for 10 min. The urine supernatant was isolated and mixed with 12 ml of acetone and incubated for a minimum of 2 hours at -20°C to allow protein precipitation. Samples were centrifuged at 3000x g for 60 min and the supernatant discarded. The protein pellet was then resuspended in 100 μl of 8M Urea and a second precipitation step was performed to increase protein yield and to discard residual contaminants using chloroform and methanol. 400 μl methanol was added to the protein solution and vortexed extensively. Subsequently, 200 μl of chloroform and 300 μl of water was added with vortexing steps in between. The mixture was centrifuged at 10000x $g$ for 15 min at room temperature to ensure phase separation. Following centrifugation, the top aqueous layer was removed leaving the lower chloroform fraction and white protein interface. To the remaining mixture, 400 μl of methanol was added followed by vortexing and centrifugation at 10000x g for 5 min. Supernatant was removed and the methanol wash was repeated twice. After the last methanol wash, the pellet was dried in a speed vacuum and resuspended in 8M Urea.

#### 1.4 Enzymatic digestion of precipitated proteins

Precipitated proteins were reduced in 10 mM DTT (Roche) at 37°C for 1 hour and diluted to 4M Urea. Following reduction, proteins were subsequently alkylated with 20 mM Indole-3-acetic acid (Merck) for 1h at room temperature in the dark and diluted to 2M Urea.

Following reduction and alkylation, proteins were initially enzymatically digested with LysC (Lysyl Endopeptidase®, Mass Spectrometry Grade, FUJIFILM) at 37°C for 2h according to the manufacturer's instructions. Lastly, peptides were further digested with trypsin (Trypsin Gold, Mass Spectrometry Grade, Promega) overnight at 37°C according to the manufacturer's instructions.

### 1.5 Bulk proteomics, Data-independent acquisition mass spectrometry

Individual peptide samples were analyzed by LC-MS/MS. The nano HPLC system used was an UltiMate 3000 nano HPLC RSLC (Thermo Scientific) equipped nano-electrospray source (CaptiveSpray source, Bruker Daltonics), coupled to a timsTOF HT mass spectrometer (Bruker Daltonics).

Peptide samples were injected on a pre-column (PepMap C18, 5mm$\times$300$\mu$m$\times$5$\mu$m, 100 Å pore size, Thermo Scientific) with 2% ACN/water (v/v) containing 0.1% TFA at a flow rate of 10$\mu$L/min for 10min. Peptides were then separated on a 25cm Aurora ULTIMATE series HPLC column equipped with an emitter (CSI, 25cm$\times$75$\mu$m ID, 1.7$\mu$m C18, IonOpticks) operating at 50°C controlled by the Column Oven PRSO-V1-BR (Sonation), using UltiMate 3000 (Thermo Scientific Dionex). The analytical column flow was run at 300nL/min with two mobile phases: water with 0.1% FA (A) and water with 80% acetonitrile and 0.08% formic acid (B). A and B were applied in linear gradients as follows (only B percentages reported): starting from 2% B: 2%-10% B in 10min, 10%-24% B in 35min, 24%-35% B in 15 min, 35%-95% B in 1min, 95% for 5 min, and finally the column was equilibrated in 2% B for next 10min (all % values are v/v; Water and ACN solvents were purchased from Thermo Fisher Scientific at LC-MS grade).

The LC system was coupled to a TIMS quadrupole time-of-flight mass spectromecter (timsTOF HT, Bruker Daltonics) and samples were measured in dia-PASEF mode. The CaptiveSpray source parameters were: 1600V capillary voltage, 3.0l/min dry gas, and 180°C dry temperature. MS data was acquired in the MS scan mode, using positive polarity, 100-1700 m/z range, mobility range was set up from 0.64-1.42 V s/cm$^2$, ramp time was set to 166 ms and the estimated cycle time was 1.52s. Collision energy was 20 eV at $1/K_0$ 0.6 V s/cm$^2$, and 80 eV at $1/K_0$ 1.6 V s/cm$^2$. Automatic calibration of ion mobility was enabled. The timsTOF HT was operated in DIA mode where 1 MS1 scan was followed by 8 DIA-PASEF frames.

### 1.6. Proteomics data analysis

DIA data was analyzed in Spectronaut 18.5 (Biognosys). Trypsin/P was specified as a proteolytic enzyme and up to 2 missed cleavages were allowed in the Pulsar direct DIA search. Dynamic mass tolerance was applied for the TOF calibration. Detected peptides were initially matched against the human UniProt database (20230710, 20 586 sequences) with common contaminants (344 sequences) and common tags (28 sequences) appended. Later, detected peptides were matched against Lipocalin 15, Serum amyloid A1, Haptoglobin and control unique sequences. Carbamidomethylation of cysteine was searched as a fixed modification, whereas oxidation of methionine and acetylation at protein N-termini were defined as variable modifications. Peptides with a length between 7 and 52 amino acids were considered and results were filtered for 1% FDR at the peptide spectrum match (PSM), Peptide and Protein Group Level. Quantification was performed as specified in Biognosys BGS Factory Default settings, grouping Peptides by Stripped Sequence, and performing protein inference using IDPicker. For normalization Cross-Run Normalization in Spectronaut was activated.

Spectronaut results were exported using Pivot Reports on the Protein and Peptide level and converted to Microsoft Excel files. Missing values were imputed with values obtained from a log-normal distribution with a mean of 30 in msReport and statistical significance of differentially expressed proteins was determined using limma-moderated Benjamini-Hochberg-corrected two-sided t-test (Smyth, Statistical applications in genetics and molecular biology, 2004, 3(1)).

### 1.7. Parallel reaction monitoring mass spectrometry (PRM-MS) Relative peptide amount determination

Before NanoLC-MS/MS analysis, final peptide amounts were determined by separating an aliquot of each sample on an LC-UV system equipped with a monolith column (Thermo scientific technical note 72602) and normalizing it to the peak area of 100 ng of Pierce HeLa protein digest standard (PN 88329; ThermoFisher Scientific).

### 1.8. NanoLC-MS/MS analysis

The nano HPLC system used was a Vanquish Neo UHPLC-System coupled to a Orbitrap Exploris 480 mass spectrometer, equipped with an Easy spray Source TNG (Thermo Fisher Scientific). Peptides were loaded onto a trap column (PepMap C18, 5 mm $\times$ 300 um ID, 5 um particles, 100 Å pore size, Thermo Fisher Scientific) by using 0.1% TFA. The trap column was switched in line with the analytical column (Double nanoViper™ PepMap C18, 500 mm $\times$ 75 um ID, 2 µm, 100 Å, Thermo Fisher Scientific). The analytical column was connected to PepSep sprayer 1 (Bruker) equipped with a 10 um ID fused silica electrospray emitter with an integrated liquid junction (Bruker, PN 1893527). Electrospray voltage was set to 2.3 kV.

The analytical column flow was run at 230nL/min, at 60 min binary gradient, with two mobile phases: water with 0.1% formic acid (A) and water with 80% acetonitrile and 0.08% formic acid (B). A and B were applied in linear gradients as follows (only B percentages reported): starting from 2% B: 2%-10% B in 10min, 10%-24% B in 35min, 24%-35% B in 15 min, 35%-95% B in 1min, 95% for 5 min, and finally the column was equilibrated in 2% B for 3 analytical column volumes at 30°C (all % values are v/v; Water and ACN solvents were purchased from Thermo Scientific Price at LC-MS grade).

The Orbitrap Exploris 480 mass spectrometer was operated by a mixed MS method which consisted of one full scan (m/z range 380-1500; 15000 resolution; target value 100%) followed by the PRM of targeted peptides from an inclusion list (isolation window 0.8 m/z; normalized collision energy (NCE) 34; 30000 resolution, AGC target 200%). Spectra of unique peptides of the proteins of interest was recorded. Per protein at least 2 unique peptides were measured. The maximum injection time was set to 125 ms. Each precursor was measured in a 5 min time window.

A scheduled PRM method (sPRM) development, data processing and manual evaluation of results was performed in Skyline (64-bit, v22.2.0.351). To derive the PRM score for each protein of interest (POI), i.e. the marker proteins, the area of each identified peptide (Peptide area, PA) was summed and divided by the sum of all areas from all identified peptides from the three normalization proteins:

$$\text{PRM score (POI)} = \frac{\sum \text{PA(POI)}}{\sum \text{PA(UMOD)} + \sum \text{PA(KLK1)} + \sum \text{PA(APOD)}}$$

### 1.9. UrineScore calculation

The UrineScore was calculated in the following way: The PRM score of the three POIs was calculated for controls, nccRCC and ccRCC patients. The 95% confidence interval (CI) of the median of the PRM scores was calculated for the three POIs for the controls. (i) Haptoglobin 95% CI of median = 0.003772 - 0.03508, (ii) SAA1 95% CI of median = 0 - 5.35E-9, (iii) LCN15 95% CI of median = 0 - 2.02E-5.

Then, for each control and patient sample, the PRM scores for each POI was compared to the corresponding proteins 95% CI of the controls. If the PRM score was higher than the upper limit of the controls 95% CI, a value of 1 was attributed. This calculation was done for all three proteins and the values added together, meaning that each control and patient sample will receive a UrineScore which is an integer between 0-3.

### 1.10. Metabolomics

Metabolites were extracted from each sample by mixing 20 µl of urine supernatants with 200 ul of methanol. Samples were subsequently dried down in a vacuum centrifuge and resuspended in 0.1% formic acid. Creatinine levels were determined in a targeted LC-MS/MS experiment. Normalized to the amount of creatinine determined, another aliquot of each extracted sample was evaporated and resuspended in 130 µl ACN:H2O (80:20). Samples were then centrifuged at 4°C for 10 min at 16000 g and transferred to a glass HPLC vial. 2 µl of all samples were pooled and used as a quality control (QC) sample. Samples were randomly assigned into the autosampler, and metabolites were separated on a iHILIC®-(P) Classic HPLC column (HILICON AB, 100 × 2.1 mm; 5 µm; 200 Å, Sweden) with a flow rate of 100 µl/min delivered through an Ultimate 3000 HPLC system (Thermo Fisher Scientific, Germany). The stepwise gradient starts at 90% A (ACN) and takes 21 min to 60% B (25 mM ammonium bicarbonate) followed by 5 min hold at 80% B and subsequent equilibration phase at 90% A with a total run time of 35 min. Sample spectra were acquired by a high-resolution tandem mass spectrometer (Q-Exactive Focus, Thermo Fisher Scientific, Germany) in full MS mode. Metabolites were ionized via electrospray ionization in polarity switching mode after HILIC separation. Ionization potential was set to +3.5/- 3.0 kV, the sheet gas flow was set to 20, and an auxiliary gas flow of 5 was used. Samples were subjected to randomized analysis, flanked by a blank and a QC sample for background correction and data normalization, respectively, occurring after every set of 8 samples. QC samples were additionally measured in data-dependent and confirmation mode to obtain MS/MS spectra for identification. The obtained data set was processed by "Compound Discoverer 3.3 SP2" (Thermo Fisher Scientific). Compounds were annotated through searching against our internal mass list database which was generated with authentic standard solutions. Additional compound annotation was conducted by searching the mzCloud database and ChemSpider hits were obtained using BioCyc, Human Metabolome Database, and KEGG databases.

### 1.11. Statistical analysis

All statistical analysis were performed in Prism 10 (GraphPad) or in RStudio (Posit). When comparing large omics datasets (TCGA transcriptomics, proteomics, or metabolomics) p values were calculated with limma-moderated Benjamini-Hochberg-corrected two-sided t-test after data processing for proteomics and metabolomics, and Benjamini-Hochberg adjusted Wald p values for TCGA data. For comparisons of individual markers between groups, the distribution of the data was either performed using Mann-Whitney U-tests (two groups) or Kruskal-Wallis test with Kruskal-Wallis test with Dunn's multiple comparisons test (three groups). The performance of receiver operating characteristic curve (ROC) was assessed with the Area under ROC method (AUC) and a p value calculated by testing the null hypothesis that the AUC is equal to 0.5. Gene Ontology analysis was performed using online portals, Enrichr for proteomics data and MetaboAnalyst for metabolomics data.

**Example 2: Transcriptomic analysis of TCGA data shows putative secreted diagnostic biomarkers for ccRCC**

Out of the three types of kidney cancer present in the cancer genome atlas (TCGA), ccRCC KIRC is by far the deadliest **(Fig. 1A).** Due to the high mortality rate compared to other kidney cancers, finding early diagnostic biomarkers is of high interest to manage and treat the disease at an early stage. ccRCC is proposed to predominantly arise from proximal tubular epithelial cells within the proximal nephrons. The proximal tubules are the main reabsorption units in the nephron and thus directly filter molecules between the blood and urine. Therefore, upregulated secreted proteins directly from the tumor microenvironment could be found at higher levels in the urine of ccRCC patients and serve as a diagnostic tool **(Fig. 1B).**

To test the feasibility of using secreted protein biomarkers to diagnose ccRCC, we performed an *in silico* screen on TCGA transcriptomics data from ccRCC (KIRC), pRCC (KIRP) and chRCC (KICH) patients **(Fig. 1C).** Differential expression analysis was performed between tumor and normal tissue for the three types of cancer and all non-secreted genes were removed. For KIRC, a very large number of genes were found to be dysregulated in the tumor microenvironment **(Fig. 1D)** and when excluding non-secreted genes and implementing a cut-off of $\log_2$ fold change > 1 a total of 615 genes remained as potential diagnostic biomarkers **(Fig. 1E).** Similarly, analysis of KIRP and KICH also revealed a large number of secreted biomarkers (454 and 317, respectively) **(Fig. 5A-D).** Interestingly, the overlap between both upregulated and downregulated genes in the different types of kidney cancer was low **(Fig. 5E-F),** indicating that secreted diagnostic biomarkers also could be cancer specific and allow discrimination between different kinds of cancer types.

**Example 3: Patient cohort characteristics**

To validate the TCGA data on the protein level, a clinical cohort with kidney cancer was recruited and urine samples collected. The cohort consisted of 22 patients with ccRCC, 9 patients with non-clear cell renal cell carcinoma (nccRCC) from which 8 patients had pRCC and 1 patient chRCC, and 12 healthy controls **(Fig. 2A).** Of note, the ccRCC patients predominantly consisted of patients with stage 1 tumors, 14 of the patients had pT1 tumors **(Fig. 2B),** allowing us to focus our study on the discovery of early-stage diagnostic biomarkers. All urine samples were centrifuged to separate the supernatant fraction from the sediment fraction. Both fractions were separately processed and analyzed with data-independent acquisition mass spectrometry (DIA-MS/MS).

ccRCC patients had the highest amounts of protein in the urine supernatant **(Fig. 6A).** However, the amount of protein found in the urine of ccRCC patients was independent of the tumor stage **(Fig. 6B).** Furthermore, for leukocytes and inflammatory damage markers (Creatinine and C-reactive protein) in the serum no differences were found between ccRCC and nccRCC patients, and between different stages of ccRCC **(Fig. 6C-D).** Lastly, we found that precipitating proteins in two steps from the urine samples (tested on supernatant) starting with an acetone precipitation followed by a chloroform:methanol precipitation yielded the highest number of unique peptides and proteins from control samples **(Fig. 2A, Fig. 7).** This method was subsequently used for all protein preparations.

**Example 4: Proteomics on urine supernatant reveal and validate diagnostic biomarkers for ccRCC**

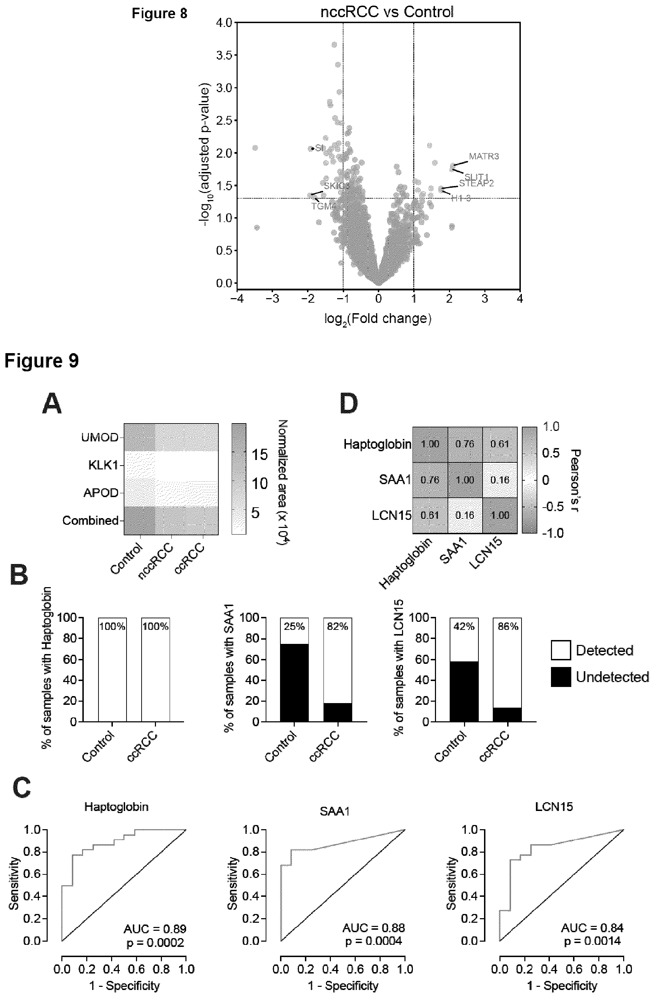

For diagnostic biomarkers, directly accessible proteins in the urine supernatant would be the preferred source to accurately assess secreted biomarkers from the tumor itself and due to the variability in sediment amounts acquired from individual patients. Urine supernatants revealed relatively weak clustering between different groups **(Fig. 2C)** indicating low global changes in the urinary proteome, but dysregulated proteins in differential expression analysis were found. When comparing ccRCC against controls, three upregulated proteins emerged as potential biomarkers: Serum amyloid A1 (SAA1), Haptoglobin (HP) and Lipocalin 15 (LCN15) which are all secreted proteins **(Fig. 2D).** In contrast, nccRCC showed a lower number of upregulated proteins and the top hits are not secreted proteins which could end up in the urine through tissue damage in the tumor microenvironment **(Fig. 8).** However, when comparing ccRCC against nccRCC we found that SAA1 and HP were also significantly upregulated in ccRCC, meaning that SAA1 and HP might also serve as discriminators between ccRCC and nccRCC **(Fig. 2E-F).** The three biomarkers (SAA1, HP, and LCN15) are all predominantly produced in different sites of the body other than the kidney - SAA1 and HP are found in the liver as acute stress response proteins and LCN15 is a transporter protein mainly found in the gastrointestinal tract (Human Protein Atlas; Uhlén et al., Science, 2015, 347(6220): 1260419). However, despite a low baseline expression in the kidney, they are all significantly upregulated in ccRCC tumors at the RNA level, and they were all hits in our TCGA in silico screen **(Fig. 2G).**

**Example 5: Parallel reaction monitoring mass spectrometry allows rapid detection of SAA1, HP and LCN15 in ccRCC urine**

Conventional bulk proteomics analysis allows discovery at the full proteome level but is not suitable for rapid diagnostics in a clinical setting due to long instrument run times and computationally intensive data analysis. Therefore, we utilized parallel reaction monitoring mass spectrometry (PRM-MS) to develop a rapid diagnostic modality for SAA1, HP and LCN15. PRM-MS is an ion monitoring technique allowing parallel high-resolution detection of all product ions from pre-selected peptides of interest, drastically reducing the run time per sample compared to bulk proteomics and other ion monitoring techniques, such as selected reaction monitoring, which are dependent on sequential ion detection (Peterson et al., Molecular & cellular proteomics, 2012, 11(11): 1475-1488).

In our PRM-MS approach, we included all detected peptides for SAA1, HP and LCN15 in bulk proteomics and all peptides from three proteins that were defined as housekeeping proteins; Uromodulin (UMOD), Kallikrein-1 (KLK1) and Apolipoprotein D (APOD). A housekeeping protein was defined as a protein which was (i) found in every sample in all three groups (Control, nccRCC and ccRCC) and (ii) had expression values which did not differ by more than one order of magnitude across all three groups **(Fig. 9A).** The peptide area is calculated for every selected peptide and the PRM score for each diagnostic protein is calculated by dividing the sum of all peptide areas from individual proteins of interest (SAA1, HP, LCN15) with the sum of all peptides from all three housekeeping proteins **(Fig. 3A).** Whereas HP was detected in all analyzed samples, SAA1 and LCN15 were predominantly detected in ccRCC samples (82% and 86%, respectively) and more rarely in controls (25% and 42%, respectively) **(Fig. 9B).** The PRM scores for SAA1, HP and LCN15 were all significantly higher in ccRCC patients compared to controls **(Fig. 3B-D).** The classification performance of each PRM score was calculated as the area under the receiver operating characteristic (ROC) curve and was evaluated to be 0.88, 0.89 and 0.84 for SAA1, HP and LCN15, respectively **(Fig. 9C).** The PRM score for Haptoglobin showed high positive correlation with both SAA1 and LCN15 (Pearson's r = 0.76 and 0.61, respectively), whereas SAA1 and LCN15 did not show any correlation with one another (r = 0.16). Lastly, all three individual PRM scores were combined into a cumulative UrineScore, detailed in the Methods section. In brief, the 95% confidence interval (CI) of the median was calculated for all PRM parameters for the control samples. Subsequently, each sample from the control, nccRCC and ccRCC group was attributed a score of 1 per protein (SAA1, HP and LCN15) which had a value which was higher than the upper limit of the control 95% CI, meaning that the UrineScore is an integer between 0-3 per control or patient. The UrineScore was the highest for the ccRCC group **(Fig. 3E)** and had a very high performance accuracy in distinguishing between control and ccRCC samples (AUC = 0.96), and also performed well between nccRCC and ccRCC samples (AUC = 0.79) **(Fig. 3F).**

**Example 6: Bulk proteomics on ccRCC urine indicates aberrant metabolism**

Gene ontology (GO) term analysis on all upregulated proteins in ccRCC urine supernatants, compared to controls, predominantly revealed metabolic dysregulation, mainly affecting lipid metabolism and function through e.g. fatty acid transport and high-density lipoparticle remodeling **(Fig. 5A).**
To derive further biological insights into how the urine in ccRCC patients indicates dysregulation in the urogenital tract, we also performed bulk proteomics analysis on the sediments from control, nccRCC patient and ccRCC patient urine samples **(Fig. 2A).** Similar to the supernatant, only weak clustering was evident in a PCA analysis **(Fig. 10A)** but multiple down- and upregulated proteins were detected between the different conditions **(Fig. 10B-D).** GO term analysis on the upregulated proteins in ccRCC, supernatants compared to controls, revealed further metabolic dysregulation. In ccRCC sediments, mitochondrial respiration and electron transport processes were upregulated **(Fig. 4B)** .

**Example 7: Lipid and mitochondrial metabolism are upregulated in ccRCC**

Having the indication that the urinary landscape in ccRCC patients hints at metabolic dysregulation, we performed full metabolomics on urine supernatants from ccRCC patients and controls **(Fig 4C).** Surprisingly, untargeted metabolomics revealed almost exclusively downregulated metabolites in ccRCC patients **(Fig 11A).** After filtering the hits further to only include metabolites from the mzCloud database and our in-house validated database, only 36 downregulated metabolites remained **(Fig 4D),** some examples are shown in **Fig. 11B.** GO term analysis on the 36 downregulated metabolites, matched against the KEGG and SMPDB databases, revealed downregulation of (i) aspartate, alanine and glutamate metabolism, (ii) glyoxylate metabolism, (iii) glutathione metabolism and (iv) carnitine synthesis **(Fig. 4E).** Combined, the metabolism related data hints at a reduction of alternative ways of entering, and the faster glyoxylate cycle way through, the citric acid cycle **(Fig. 4F).** Furthermore, reduced carnitine levels lead to a reduced breakdown of free fatty acids, meaning that there might also be an increased transport of free fatty acids, and not only cholesterol. In conclusion, our results implicate some of the first diagnostic biomarkers for ccRCC and highlight metabolic dysregulation in the urogenital tract of ccRCC patients.

**Discussion**

The invention provides and validated three diagnostic biomarkers (SAA1, HP and LCN15) in a clinical cohort. Furthermore, through PRM-MS for rapid and sensitive detection of our three proteins of interest PRM scores for SAA1, HP and LCN15 with high diagnostic power were derived. When these PRM scores were combined into an overarching UrineScore we calculated an overall model performance in an area under ROC analysis of 96% in distinguishing between healthy controls and ccRCC. Further shown distinctions are control and nccRCC; and ccRCC and nccRCC (Fig. 3E and F)

The three protein biomarkers SAA1, HP and LCN15 show a very good performance for the diagnosis of renal cancer and also a good ability to differentiate between ccRCC and nccRCC, highlighting the specificity of these markers towards ccRCC. Furthermore, the data herein suggests the occurrence of distinct molecular mechanisms underlying the different renal cell carcinomas.

Of note, the protein biomarkers were found to be highly upregulated in the ccRCC tumor microenvironment based on RNA sequencing data from TCGA. However, they are also produced in other parts of the body at high levels based on human protein atlas data. SAA1 and HP are produced as acute phase proteins in the liver, and LCN15 is produced in the gastrointestinal tract. Here, an evident upregulation in the tumor microenvironment is shown. One possible mechanism is that circulating serum proteins are found at higher levels in the urine of ccRCC patients due to tumor-induced tissue damage of the filtration unit of the kidney, effectively increasing the leakiness of serum proteins into the urine which is found in ccRCC associated hematuria.

Further, aberrant metabolism was uncovered throughout the urogenital tract of ccRCC patients as indicated by urinary proteomics and metabolomics. The present data suggests a reduction of alternative pathways around, and through, the citric acid cycle so that more substrates are free to be utilized in mitochondrial respiration. Furthermore, ccRCC is known to have a lipid rich cytoplasm. A reduction in carnitine synthesis and increased lipid transport was discovered. Although the present data comes from the urine and not directly from the tumor, one potential explanation for the increased lipid accumulation in ccRCC tumors could be a reduction of carnitine, which is essential for the breakdown of free fatty acids and potentially lipid transport to the tumor microenvironment, and not only increased lipid transport through the reverse cholesterol pathway in which cholesterol is recycled to the liver.

**Claims**

1. A method of detecting renal cancer markers in a biological sample from a patient suspected of or to be evaluated for having renal cancer comprising detecting one or more marker proteins selected from Lipocalin 15, Serum amyloid A1, and Haptoglobin, in the biological sample.

2. The method of claim 1, wherein the biological sample comprises urine.

3. The method of claim 1 or 2, wherein detecting one or more marker proteins comprises a determination of amounts to the one or more marker proteins by mass spectrometry or affinity binding, preferably by determining a total signal intensity detected by mass spectrometry associated with the one or more marker proteins.

4. The method of any one of claims 1 to 3, wherein detecting one or more marker proteins comprises fragmenting the one or more marker proteins to protein fragments and detecting the protein fragments by mass spectrometry.

5. The method of any one of claims 1 to 4, wherein an amount of the one or more marker proteins in the biological sample is determined and compared to the amount of the one or more marker proteins in a healthy control sample.

6. The method of any one of claims 1 to 5, wherein one or more further proteins that are not indicative of renal cancer are determined as control proteins in the biological sample, preferably wherein the further proteins are renal housekeeping proteins, in particular preferred wherein the further proteins that are not indicative of renal cancer are selected from Uromodulin, Kallikrein-1, and Apolipoprotein D.

7. The method of claims 6, comprising determining the amount of the control proteins in the biological sample, preferably wherein the relation of the amount of the one or more marker proteins in the biological sample to the amount of the one or more further proteins in the biological sample is determined.

8. The method of any one of claims 1 to 7, wherein the one or more marker proteins are Lipocalin 15, Serum amyloid A1, and Haptoglobin.

9. The method of any one of claims 1 to 8, wherein the biological sample is a urine sample, the method further comprising precipitating a protein fraction of the urine sample, removing a supernatant from the protein fraction, resuspending the protein fraction, fragmenting the one or more marker proteins to protein fragments, chromatographically separating protein fragments, detecting protein fragments by mass spectrometry.

10. The method of any one of claims 4 or 9, wherein detecting protein fragments by mass spectrometry comprises the determination of an amount of the protein fragments.

11. The method of any one of claims 4, 9 or 10, wherein fragmenting the one or more marker proteins comprises enzymatic digestion, preferably with an endopeptidase.

12. The method of claim 10 or claim 11 in combination with claim 10, further comprising determining the amount of protein fragments of one or more further proteins that are not indicative of renal cancer in the biological sample; preferably wherein the further proteins are renal housekeeping proteins; especially preferred wherein the further proteins that are not indicative of renal cancer are selected from Uromodulin, Kallikrein-1, and Apolipoprotein D.

13. The method of claim 12, further comprising determining if a patient has renal cancer or if a patient is suspected of having renal cancer or determining the type of renal cancer that a patient has, by comparing the amount of the protein fragments of the one or more marker proteins to the amount of the one or more further proteins.

14. The method of any one of claims 1 to 13, wherein renal cancer is clear cell renal cell carcinoma.

15. A kit comprising marker proteins or marker protein fragments, wherein the marker proteins are Lipocalin 15, Serum amyloid A1, and Haptoglobin; and wherein the marker protein fragments comprise fragments from each of marker proteins Lipocalin 15, Serum amyloid A1, and Haptoglobin, and wherein
fragments of Lipocalin 15 are selected from fragments with amino acid sequences DHLSMSTRA (SEQ ID NO: 1), VGSEGHFR (SEQ ID NO: 2), VPALGYLDVR (SEQ ID NO: 3), TQDVSPQALK (SEQ ID NO: 4), fragments of Serum amyloid A1 are selected from fragments with amino acid sequences GPGGVWAAEAISDAR (SEQ ID NO: 5), FFGHGAEDSLADQAANEWGR (SEQ ID NO: 6),

fragments of Haptoglobin are selected from fragments with amino acid sequences NPANPVQR (SEQ ID NO: 7), GSFPWQAK (SEQ ID NO: 8), DIAPTLTLYVGK (SEQ ID NO: 9), VGYVSGWGR (SEQ ID NO: 10), HYEGSTVPEK (SEQ ID NO: 11), SCAVAEYGVYVK (SEQ ID NO: 12), VTSIQDWVQK (SEQ ID NO: 13);
optionally wherein the kit further comprises one or more further proteins or further protein fragments, wherein the further proteins are selected from Uromodulin, Kallikrein-1, and Apolipoprotein D; and wherein the further protein fragments comprise fragments from one or more of further proteins Uromodulin, Kallikrein-1, and Apolipoprotein D, and wherein
fragments of Uromodulin are selected from fragments with amino acid sequences FVGQGGAR (SEQ ID NO: 14), DWVSWTPAR (SEQ ID NO: 15), DGPCGTVLTR (SEQ ID NO: 16), INFACSYPLDMK (SEQ ID NO: 17), SGSVIDQSR (SEQ ID NO: 18), VLNLGPITR (SEQ ID NO: 19),
fragments of Kallikrein-1 are selected from fragments with amino acid sequences QWVLTAAHCISDNYQLWLGR (SEQ ID NO: 20), QADEDYSHDLMLLR (SEQ ID NO: 21), LTEPADTITDAVK (SEQ ID NO: 22), ILPNDECK (SEQ ID NO: 23),
fragments of Apolipoprotein D are selected from fragments with amino acid sequences CPNPPVQENFDVNK (SEQ ID NO: 24), IPTTFENGR (SEQ ID NO: 25), NILTSNNIDVK (SEQ ID NO: 26), MTVTDQVNCPK (SEQ ID NO: 27).

# Figure 1

**A**

KIRC  KIRP  KICH

KIRC vs KIRP: p = 0.0013
KIRC vs KICH: p < 0.0001

**B**

Primary ccRCC tumor

Urine with ccRCC biomarkers

**C** *In silico* screening pipeline

KIRC
KIRP
KICH

TCGA raw sequencing data download

Differential expression analysis between tumor and normal tissue

Secretion prediction:
1. The human secretome (PMID: 31772123)
2. UniProt

**D** All genes

**E** Secreted genes

n = 615
(Log$_2$FC ≥ 1; adj. p ≤ 0.05)

HP
SAA1
LCN15

n = 293
(Log$_2$FC ≤ -1; adj. p ≤ 0.05)

# Figure 2

# Figure 3

# Figure 4

**A** Top 5 upregulated Biological Processes in supernatant

**B** Top 5 upregulated Biological Processes in sediment

**C**

**D** mzCloud and Internal database filtered hits

**E** Downregulated Biological Processes

**F**

# Figure 5

# Figure 6

# Figure 7

**Figure 8**

nccRCC vs Control

**Figure 9**

## Figure 10

**A**

**B**

**C**

**D**

Figure 11

Europäisches
Patentamt

European
Patent Office

Office européen
des brevets

# EUROPEAN SEARCH REPORT

**Application Number**

EP 24 19 0080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | YANG YIREN ET AL: "Excavation of diagnostic biomarkers and construction of prognostic model for clear cell renal cell carcinoma based on urine proteomics", FRONTIERS IN ONCOLOGY, vol. 13, 16 May 2023 (2023-05-16), XP093250581, ISSN: 2234-943X, DOI: 10.3389/fonc.2023.1170567 * abstract; page 02, left-hand column, last paragraph; section 3.2 * * Relevant for assessing the lack of unity of invention. * | 1-15 | INV. G01N33/574 G01N33/68 |
| A | SANDIM VANESSA ET AL: "Proteomic analysis reveals differentially secreted proteins in the urine from patients with clear cell renal cell carcinoma", UROLOGIC ONCOLOGY: SEMINARS AND ORIGINAL INVESTIGATIONS, ELSEVIER, AMSTERDAM, NL, vol. 34, no. 1, 26 September 2015 (2015-09-26), XP029335087, ISSN: 1078-1439, DOI: 10.1016/J.UROLONC.2015.07.016 * abstract; page 5.e12, left-hand column - right-hand column, bridging paragraph; page 5.e20; figure 6 * * Relevant for assessing the lack of unity of invention. * | 1-15 | |
| | | | **TECHNICAL FIELDS SEARCHED (IPC)** |
| | | | G01N |
| X | WO 2024/119178 A1 (NOVELNA INC [US]) 6 June 2024 (2024-06-06) * paragraphs [0011], [00166]; claims 215, 271-277; Table 2008 (page 203); Table 2008.C.1 (pages 205-206); page 99, example 3 * | 1,3-7, 10-13 | |

-/--

The present search report has been drawn up for all claims

| Place of search | Date of completion of the search | Examiner |
|---|---|---|
| The Hague | 18 February 2025 | Adida, Anne |

EPO FORM 1503 03.82 (P04C01)

Europäisches
Patentamt

European
Patent Office

Office européen
des brevets

# EUROPEAN SEARCH REPORT

**Application Number**

EP 24 19 0080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PAN JIANBO ET AL: "Integration of IgA and IgG Autoantigens Improves Performance of Biomarker Panels for Early Diagnosis of Lung Cancer", MOLECULAR & CELLULAR PROTEOMICS, vol. 19, no. 3, 1 March 2020 (2020-03-01), pages 490-500, XP093251093, US ISSN: 1535-9476, DOI: 10.1074/mcp.RA119.001905 Retrieved from the Internet: URL:https://pmc.ncbi.nlm.nih.gov/articles/ PMC7050113/pdf/zns490.pdf> * section "HuProt Arrays and Serum Profiling Assays" * | 15 | |
| L | -& Cdi Labs: "HuProt v3.0 Full Content", , 17 February 2025 (2025-02-17), pages 1-4, XP093251151, Retrieved from the Internet: URL:https://www.cdilabs.com/content/litera tures/huprot-v3-0 * This document discloses the link to the CSV file comprising the full content of the HuProt v3.0 array, used in Pan et al. * * the whole document * | 15 | TECHNICAL FIELDS SEARCHED (IPC) |

-----

-/--

The present search report has been drawn up for all claims

| Place of search | Date of completion of the search | Examiner |
|---|---|---|
| The Hague | 18 February 2025 | Adida, Anne |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................................
& : member of the same patent family, corresponding
document

EPO FORM 1503 03.82 (P04C01)

Europäisches Patentamt

European Patent Office

Office européen des brevets

**EUROPEAN SEARCH REPORT**

Application Number

EP 24 19 0080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Jonsson Gustav ET AL: "Urinary multi-omics reveal non-invasive diagnostic biomarkers in clear cell renal cell carcinoma", bioRxiv, 13 August 2024 (2024-08-13), pages 1-41, XP093250595, DOI: 10.1101/2024.08.12.607453 Retrieved from the Internet: URL:https://www.biorxiv.org/content/biorxiv/early/2024/08/13/2024.08.12.607453.full.pdf * the whole document * | | |
| A | WO 2018/144834 A1 (UNIV DUKE [US]) 9 August 2018 (2018-08-09) * page 51, Table S4, first line; sequence 41 * | 15 | |

TECHNICAL FIELDS SEARCHED (IPC)

The present search report has been drawn up for all claims

| Place of search | Date of completion of the search | Examiner |
|---|---|---|
| The Hague | 18 February 2025 | Adida, Anne |

EPO FORM 1503 03.82 (P04C01)

page 3 of 3

Europäisches
Patentamt

European
Patent Office

Office européen
des brevets

**Application Number**

**EP 24 19 0080**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

The Search Division considers that the present European patent application does not comply with the requirements of unity of invention and relates to several inventions or groups of inventions, namely:

1. claims: 1-15(partially)

   Method of detecting renal cancer marker proteins in a biological sample from a patient suspected of or to be evaluated for having renal cancer and kit comprising marker proteins or marker protein fragments, wherein the marker proteins comprise lipocalin 15.
   
   ---

2. claims: 1-15(partially)

   Method of detecting renal cancer marker proteins in a biological sample from a patient suspected of or to be evaluated for having renal cancer and kit comprising marker proteins or marker protein fragments, wherein the marker proteins comprise serum amyloid A1.
   
   ---

3. claims: 1-15(partially)

   Method of detecting renal cancer marker proteins in a biological sample from a patient suspected of or to be evaluated for having renal cancer and kit comprising marker proteins or marker protein fragments, wherein the marker proteins comprise Haptoglobin.
   
   ---

## ANNEX TO THE EUROPEAN SEARCH REPORT
## ON EUROPEAN PATENT APPLICATION NO.

EP 24 19 0080

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2025

| Patent document cited in search report | Publication date | Patent family member(s) | | Publication date |
|---|---|---|---|---|
| WO 2024119178 A1 | 06-06-2024 | NONE | | |
| WO 2018144834 A1 | 09-08-2018 | US | 2019376969 A1 | 12-12-2019 |
| | | WO | 2018144834 A1 | 09-08-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **DIANA et al.** *World journal of urology*, 2023, vol. 41 (4), 929-940
- **SATO et al.** *Nature genetics*, 2013, vol. 45 (8), 860-867
- **HAKIMI et al.** *European urology*, 2013, vol. 63 (5), 848-854
- **SANDIM E et al.** *Urologic Oncology: Seminars and Original In-vestigations*, 2016, vol. 34 (1), 5.e11-5.e25
- **CHINELLO et al.** *Journal of proteomics*, 2019, vol. 191, 29-37
- **YANG et al.** *Frontiers in Oncology*, 2023, vol. 13, 1170567
- **DI MEO et al.** *International journal of cancer*, 2020, vol. 146 (8), 2315-2325
- **GRAY ; HARRIS**. *American family physician*, 2019, vol. 99 (3), 179-184
- **NAIK et al.** *Ther Adv Urol*, 2024, vol. 16, 1-17
- **BURG et al.** *American Journal of Physiology-Legacy Content*, 1976, vol. 231 (2), 627-637
- **UHLÉN et al.** *Science*, 2015, vol. 347 (6220), 1260419, www.proteinatlas.org
- **PETERSON et al.** *Molecular & Cellular Proteomics*, 2012, vol. 11 (11), 1475-1488
- **PETERSON et al.** *Molecular & cellular proteomics*, 2012, vol. 11 (11), 1475-1488
- **UHLÉN et al.** *Science signaling*, 2019, vol. 12 (609), eaaz0274
- **SMYTH**. *Statistical applications in genetics and molecular biology*, 2004, vol. 3 (1)
- **UHLÉN et al.** *Science*, 2015, vol. 347 (6220), 1260419